(19) Europäisches Patentamt
European Patent Office
Office européen des brevets

(11) Veröffentlichungsnummer : **0 160 256**
**B1**

(12) # EUROPÄISCHE PATENTSCHRIFT

(45) Veröffentlichungstag der Patentschrift : **28.09.88**

(51) Int. Cl.⁴ : **C 07 D307/88**, C 07 D405/04, C 07 D409/04, A 61 K 31/365, A 61 K 31/38

(21) Anmeldenummer : **85104820.7**

(22) Anmeldetag : **20.04.85**

(54) **Annellierte Furanone, Verfahren zu ihrer Herstellung und ihre Verwendung in Arzneimitteln.**

(30) Priorität : **03.05.84 DE 3416293**

(43) Veröffentlichungstag der Anmeldung : **06.11.85 Patentblatt 85/45**

(45) Bekanntmachung des Hinweises auf die Patenterteilung : **28.09.88 Patentblatt 88/39**

(84) Benannte Vertragsstaaten : **AT BE CH DE FR GB IT LI NL SE**

(56) Entgegenhaltungen : **US-A- 3 702 853**

(73) Patentinhaber : **BAYER AG**
**Konzernverwaltung RP Patentabteilung**
**D-5090 Leverkusen 1 Bayerwerk (DE)**

(72) Erfinder : **Goldmann, Siegfried, Dr.**
**Am Osterholz 91**
**D-5600 Wuppertal 1 (DE)**
Erfinder : **Schramm, Matthias, Dr.**
**Paffrather Strasse 38**
**D-5000 Köln 80 (DE)**
Erfinder : **Thomas, Günter, Dr.**
**Claudiusweg 9**
**D-5600 Wuppertal 1 (DE)**
Erfinder : **Gross, Rainer, Dr.**
**Platzhoffstrasse 23**
**D-5600 Wuppertal 1 (DE)**

**EP 0 160 256 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue annellierte Furanone, mehrere Verfahren zu ihrer Herstellung, sowie ihre Verwendung in Arzneimitteln, insbesondere in kreislaufbeeinflussenden Arzneimitteln.

Die neuen Furanone sind durch folgende allgemeine Formel (I) gekennzeichnet.

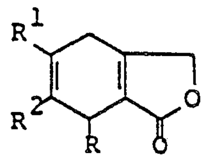

(I)

worin

R für Phenyl, Naphthyl, Pyridyl oder Thiochromenyl steht, wobei der Phenylrest gegebenenfalls durch 1 bis 3 gleiche oder verschiedene Substituenten aus der Gruppe Alkyl mit 1 bis 4 Kohlenstoffatomen, Fluor, Chlor, Nitro, Trifluormethyl, Alkoxy mit 1 bis 4 Kohlenstoffatomen, Hydroxy, Amino, Dialkylamino mit jeweils 1 bis 4 C-Atomen, Cyano, Carboxy, Carbalkoxy, Phenyl, Benzyl, Benzyloxy oder Benzylthio substituiert sein können, wobei die 4 letztgenannten Substituenten ihrerseits gegebenenfalls 1 oder 2 gleiche oder verschiedene Substituenten aus der Gruppe Alkyl, Alkoxy, Alkylthio mit jeweils 1 bis 4 C-Atomen, Fluor, Chlor, Cyano, Hydroxy, Trifluormethyl oder Nitro tragen,

$R^1$ für Hydroxyl, NH-Alkyl mit 1 bis 4 C-Atomen oder Dialkylamin mit jeweils 1 bis 4 C-Atomen in den Alkylgruppen steht und

$R^2$ für den Rest $COOR^3$ steht, wobei $R^3$ für einen geradkettigen, verzweigten oder cyclischen Alkyl- oder Alkenylrest mit bis zu 12 C-Atomen steht, der gegebenenfalls substituiert ist durch Alkoxy oder Alkylthio mit jeweils 1 bis 4 C-Atomen, Fluor, Chlor, Cyano, Hydroxy, Amino, Alkylamino oder Dialkylamino mit jeweils 1 bis 4 C-Atomen je Alkylgruppe, Nitro, Phenyl oder Pyridyl steht,

sowie ihre physiologisch unbedenklichen Salze.

Die Verbindungen der allgemeinen Formel (I), in denen $R^1 \neq OH$ ist, können hergestellt werden, indem man

A) Aldehyde der allgemeinen Formel (II)

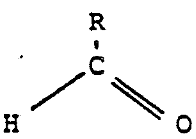

(II)

in der R die oben angegebene Bedeutung hat,
mit Verbindungen der allgemeinen Formel (III)

$$HC(R_2) = C(R_1) - CH_3 \qquad (III)$$

in der $R_1$ und $R_2$ die oben angegebenen Bedeutungen haben
und Verbindungen der allgemeinen Formel (IV)

$$R^4\text{—OCC—}CH_2\text{—CO—}CH_2\text{—O—}R^5 \qquad (IV)$$

in der

$R^4$ ein Alkylrest (1 bis 10 C-Atome) und

$R^5$ eine Schutzgruppe für eine OH oder SH-Gruppe darstellt,

in inerten organischen Lösungsmitteln bei Temperaturen zwischen 0 °C und 120 °C umsetzt und anschließend die Schutzgruppe $R^5$ abspaltet, wobei Lactonisierung eintritt, oder

B) Benzylidenverbindungen der allgemeinen Formel (V)

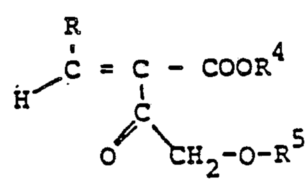

(V)

in der R, $R^4$ und $R^5$ die oben angegebenen Bedeutungen haben,
mit Verbindungen der allgemeinen Formel (III),
in der $R^1$ und $R^2$ die oben angegebenen Bedeutungen haben,
umsetzt und anschließend die Schutzgruppe $R^5$ entfernt, wobei Lactonisierung eintritt, oder

C) Benzylidenverbindungen der allgemeinen Formel (VI)

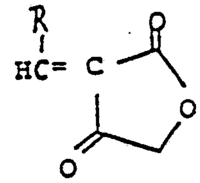

(VI)

in der R die oben angegebene Bedeutung hat,
mit Verbindungen der allgemeinen Formel (III),
in der $R^1$ und $R^2$ die oben angegebene Bedeutung haben, analog A) umsetzt.

Die Verbindungen der allgemeinen Formel (I), in denen $R^1 = OH$ darstellt, können hergestellt werden, indem man Verbindungen der allgemeinen Formel (I), in denen $R^1 \neq OH$ ist und die nach den Verfahrensvarianten A, B oder C hergestellt wurden, mit wäßrigen Säuren in Anwesenheit von inerten organischen Lösungsmitteln umsetzt.

Die Reaktionsschritte des erfindungsgemäßen Verfahrens zur Darstellung von (I) ($R^1 = OH$) können sowohl als Eintopfreaktion ohne Isolierung der hierbei entstehenden Zwischenprodukte oder in getrennten Reaktionsstufen unter Isolierung der Zwischenstufen (I) ($R^1 \neq OH$) durchgeführt werden.

Das Verfahren C wird bevorzugt, wenn Verbindungen der Struktur (I) mit $R^1 \neq OH$ erhalten werden sollen.

Die Verfahren A und B mit anschließendem Austausch der Aminogruppe durch die OH-Gruppe sind als Eintopfreaktionen bevorzugt, insbesondere zur Synthese von (I) wenn $R^1 = OH$.

Die Verbindungen der allgemeinen Formel (III) sind bekannt oder können nach bekannten Verfahren hergestellt werden (J. Am. Chem. Soc. 67, 1017 (1945)).

Die Verbindungen der allgemeinen Formel (IV) sind bekannt oder können nach bekannten Verfahren hergestellt werden (Tetrahedron 34, 1543 (1978)).

Die Verbindungen der allgemeinen Formel (V) sind bekannt oder können nach bekannten Verfahren hergestellt werden (z. B. J. Am. Chem. Soc. 66, 1933 (1944)).

Die Verbindungen der allgemeinen Formel (VI) sind bekannt oder können nach bekannten Verfahren hergestellt werden (J. Heterocycl. Chem. 20 (1983), 787, J. org. Chem. 43 (1978), 1541 oder Z. Chem. 10, 341 (1970)).

Bei der Durchführung der Verfahrensvariante A setzt man als inerte organische Lösungsmittel vorzugsweise Alkohole wie z. B. Ethanol oder tert. Butanol und Carbonsäuren wie z. B. Essigsäure, oder Propionsäure ein.

Als Reaktionstemperatur nimmt man vorzugsweise Temperaturen zwischen 20 und 100 °C, insbesondere die Siedetemperatur des verwendeten Lösungsmittels.

Die Abspaltung der Schutzgruppe $R^5$ erfolgt vorzugsweise mit geeigneten Abspaltungsagentien wie z. B. den üblichen organischen oder anorganischen Säuren oder organischen und anorganischen Basen.

Als Schutzgruppen ($R^5$) seien vorzugsweise genannt : Acylreste wie z. B. Acetyl ; Trialkylsilylreste oder der tert. Butylrest.

Falls nicht ausdrücklich anders angegeben, gelten auch für die Verfahrensvarianten B und C die genannten vorzugsweisen Reaktionsbedingungen und Reaktionsmedien.

Die Überführung der Verbindung der allgemeinen Formel (I), in denen $R^1$ nicht Hydroxyl bedeutet, in solche Verbindungen, in denen $R^1$ Hydroxyl bedeutet, durch einen nachträglichen Reaktionsschritt, erfolgt in üblicher Weise, vorzugsweise durch Umsetzung mit anorganischen Säuren wie Salzsäure oder Schwefelsäure bei gleichzeitiger Anwesenheit von inerten organischen Lösungsmitteln wie Alkoholen, z. B. Ethanol, Propanol oder tert. Butanol ; Ethern wie Diethylether, Tetrahydrofuran oder Dioxan. Diese nachträgliche Einführung der Hydroxylgruppe erfolgt vorzugsweise bei Temperaturen zwischen 10 und 150 °C, vorzugsweise bei 20 bis 100 °C, insbesondere bei der Siedetemperatur des Lösungsmittels.

Die erfindungsgemäßen Verbindungen zeigen ein nicht vorhersehbares und wertvolles pharmakologisches Wirkungsspektrum. Sie können als Cardiotonika zur Verbesserung der Herzkontraktilität dienen. Darüber hinaus können sie dadurch, daß sie den $Ca^{++}$-Einstrom in die Zelle erhöhen, als Antihypotonica, zur Senkung von Blutzucker, zur Abschwellung von Schleimhäuten und zur Beeinflussung des Salz- und/oder Flüssigkeitshaushaltes eingesetzt werden.

Die erfindungsgemäßen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerten, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Falle der Benutzung von Wasser als Verdünnungsmittel gegebenenfalls

organische Lösungsmittel als Hilfslösungsmittel verwendet werden können.

Als Hilfsstoffe seien beispielsweise aufgeführt :

Wasser, nichttoxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum Kreide), synthetische Gesteinsmehle (z. B. hochdisperse Kieselsäure, Silikate), Zucker (z. B. Rohr-, Milch- und Traubenzucker), Emulgiermitteln (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außen den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,0001 bis 1 mg/kg, vorzugsweise etwa 0,001 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation, größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele

1)

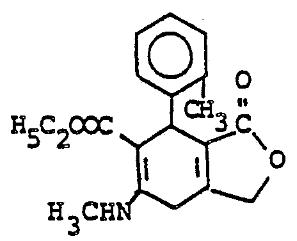

7-(2-Methylphenyl)-5-methylamino-1-oxo-1,3,4,7-tetrahydrobenzo [c] furan-6-carbonsäureethylester

(Verfahren C)

20 mMol 3-(2-Methylbenzyliden)-furan-2,4(3H,5H)-dion wurden in 50 ml Ethanol mit 20 mMol 2-Methylaminocrotonsäureethylester über Nacht am Rückfluß gekocht, eingeengt und mit Toluol/Essigester an Kieselgel chromatographiert.

Fp. : 175-181 °C.

2)

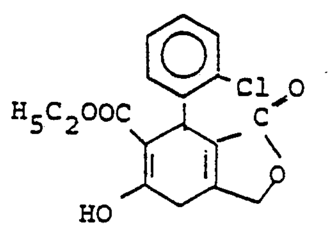

7-(2-Chlorphenyl)-5-hydroxy-1-oxo-1,3,4,7-tetrahydrobenzo [c] furan-6-carbonsäureethylester

(Verfahren B)

20 mMol 4-Acetoxy-2-(2-chlorbenzyliden)-3-oxo-butancarbonsäureethylester wurden zusammen mit 20 mMol 3-Butylamino-crotonsäureethylester über Nacht am Rückfluß gekocht, anschließend mit 50 ml konz. HCl und 10 ml Wasser versetzt und eine weitere Stunde gekocht. Nach Abkühlung wird abgesaugt.

Fp. : 181-183 °C.

3) 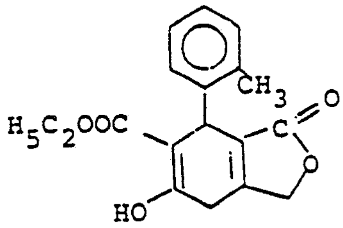

5-Hydroxy-7-(2-methylphenyl)-1-oxo-1,3,4,7-tetrahydrobenzo [c] furan-6-carbonsäureethylester

a) (Verfahren A, ohne Isolierung der Zwischenverbindung)

50 mMol 2-Methylbenzaldehyd, 50 mMol 3-Butylamino-crotonsäureethylester und 50 mMol 4-Acetoxy-3-oxo-butancarbonsäureethylester wurden zusammen in 100 ml Ethanol über Nacht am Rückfluß gekocht, anschließend mit 10 ml konz. HCl und 20 ml Wasser versetzt und eine weitere Stunde gekocht. Es wird aus Ethanol umkristallisiert.

Fp. : 145-150 °C.

b) Darstellung über die Zwischenverbindung aus Beispiel 1

10 mMol 7-(2-Methylphenyl)-5-methylamino-1-oxo-1,3,4,7-tetrahydrobenzo [c] furan-6-carbonsäureethylester werden in 50 ml THF gelöst, mit 3 ml konz. HCl und 5 ml Wasser versetzt, 1 Stunde gekocht und eingeengt. Substanz ist identisch mit der aus Beispiel 3a).

Analog Beispiel 3a wurden hergestellt :

4) 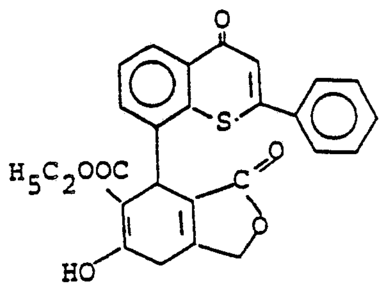

5-Hydroxy-7-(4-oxo-2-phenyl-4H-thiochromen-8-yl)-1-oxo-1,3,4,7-tetrahydrobenzo [c] furan-6-carbonsäureethylester

Fp. : 223-226 °C.

5) 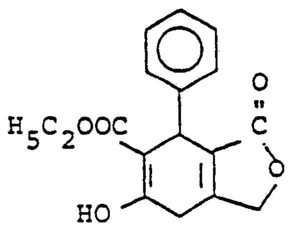

5-Hydroxy-1-oxo-7-phenyl-1,3,4,7-tetrahydrobenzo [c] furan-6-carbonsäureethylester

Fp. : 153-155 °C.

6) 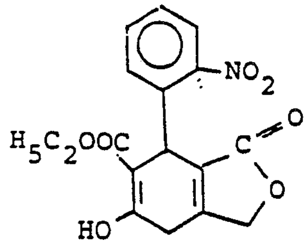

5-Hydroxy-7-(2-nitrophenyl)-1-oxo-1,3,4,7-tetrahydrobenzo [c] furan-6-carbonsäureethylester

Fp. : 183-186 °C.

7) 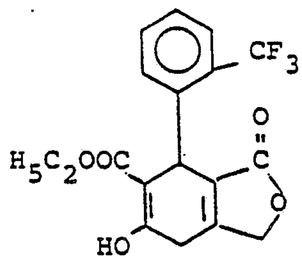

5-Hydroxy-1-oxo-7-(2-trifluormethylphenyl)-1,3,4,7-tetrahydrobenzo [c] furan-6-carbonsäureethylester

Fp. : 150 °C.

8) 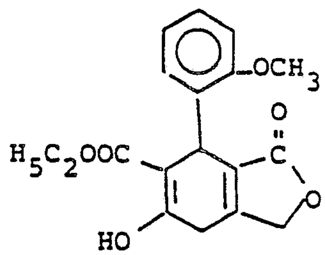

5-Hydroxy-7-(2-methoxyphenyl)-1-oxo-1,3,4,7-tetrahydrobenzo [c] furan-6-carbonsäureethylester

Fp. : 169-171 °C.

9) 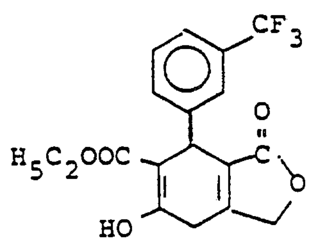

5-Hydroxy-1-oxo-7-(3-trifluormethylphenyl)-1,3,4,7-tetrahydrobenzo [c] furan-6-carbonsäureethylester

Fp. : 149-150 °C.

10) 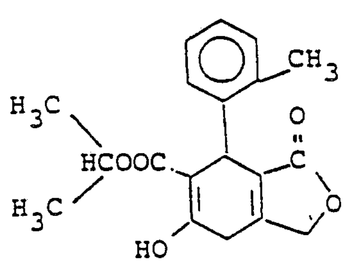

5-Hydroxy-7-(2-methylphenyl)-1-oxo-1,3,4,7-tetrahydrobenzo [c] furan-6-carbonsäureisopropylester

Fp. : 155-157 °C.

11)

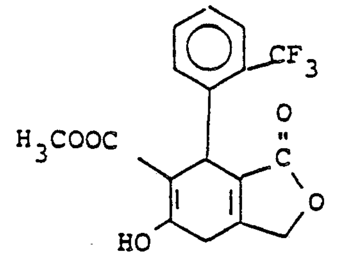

5-Hydroxy-1-oxo-7-(2-trifluormethylphenyl)-1,3,4,7-tetrahydrobenzo [c] furan-6-carbonsäuremethylester

Fp. : 156-159 °C.

12)

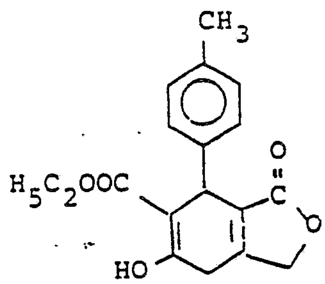

5-Hydroxy-7-(4-methylphenyl)-1-oxo-1,3,4,7-tetrahydrobenzo [c] furan-6-carbonsäureethylester

Fp. : 142-148 °C.

13)

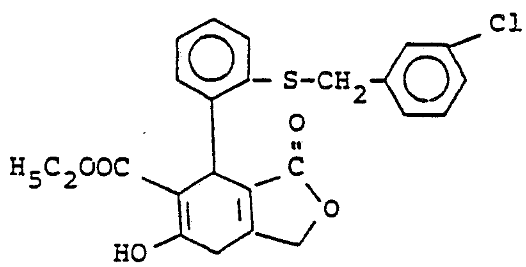

7-(2-[3-Chlorbenzylthio]-phenyl)-5-hydroxy-1-oxo-1,3,4,7-tetrahydrobenzo [c] furan-6-carbonsäureethylester

Fp. : 155-160 °C.

14)

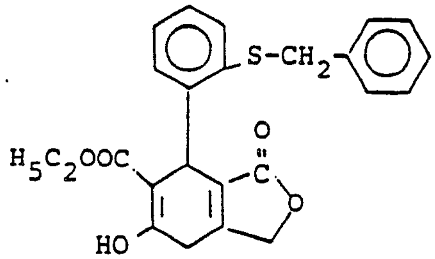

7-(2-Benzylthiophenyl)-5-hydroxy-1-oxo-1,3,4,7-tetrahydrobenzo [c] furan-6-carbonsäureethylester

Fp. : 144-147 °C.

15)

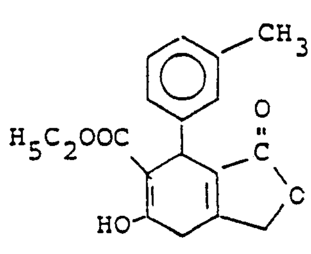

5-Hydroxy-7-(3-methylphenyl)-1-oxo-1,3,4,7-tetrahydrobenzo [c] furan-6-carbonsäureethylester

Fp. : 163-166 °C.

16) 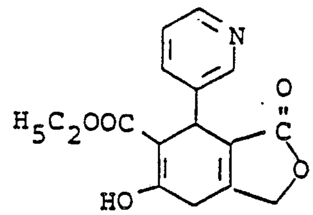

5-Hydroxy-1-oxo-7-(3-pyridyl)-1,3,4,7-tetrahydrobenzo [c] furan-6-carbonsäureethylester

Fp. : 172-175 °C.

17) 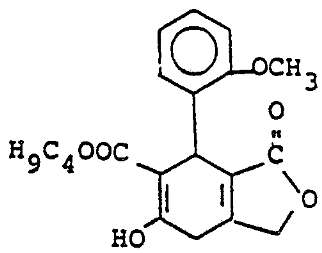

5-Hydroxy-1-oxo-7-(2-methoxyphenyl)-1,3,4,7-tetrahydrobenzo [c] furan-6-carbonsäurebutylester

Fp. : 66-69 °C.

18) 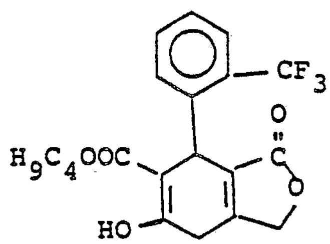

5-Hydroxy-1-oxo-7-(2-trifluormethylphenyl)-1,3,4,7-tetrahydrobenzo [c] furan-6-carbonsäurebutylester

Fp. : 122-127 °C.

19) 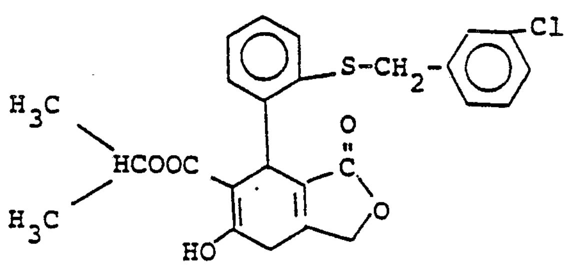

7-(4-[3-Chlorbenzylthio]-phenyl)-5-hydroxy-1-oxo-1,3,4,7-tetrahydrobenzo [c] furan-6-carbonsäureisopropylester

Fp. : 111-115 °C.

20) 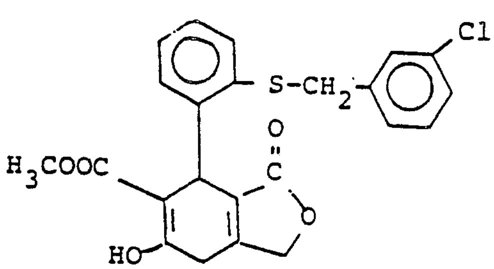

7-(4-[3-Chlorbenzylthio]-phenyl)-5-hydroxy-1-oxo-1,3,4,7-tetrahydrobenzo [c] furan-6-carbonsäuremethylester

Fp. : 168-171 °C.

21)

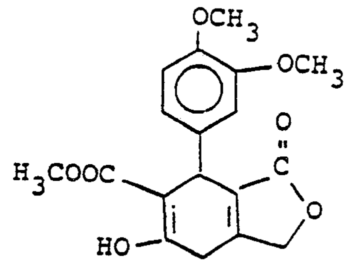

7-(3,4-Dimethoxyphenyl)-5-hydroxy-1-oxo-1,3,4,7-tetrahydrobenzo [c] furan-6-carbonsäuremethylester

Fp. : 158-160 °C.

22)

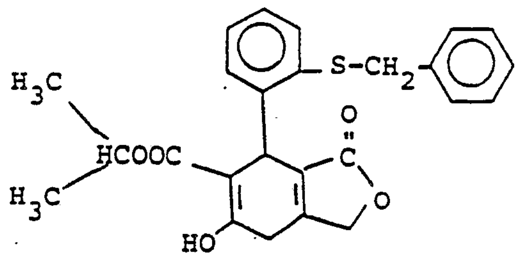

7-(2-Benzylthiophenyl)-5-hydroxy-1-oxo-1,3,4,7-tetrahydrobenzo [c] furan-6-carbonsäuremethylester

Fp. : 149-154 °C.

23)

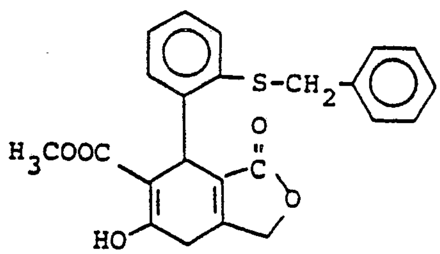

7-(2-Benzylthiophenyl)-5-hydroxy-1-oxo-1,3,4,7-tetrahydrobenzo [c] furan-6-carbonsäuremethylester

Fp. : 149-154 °C.

24)

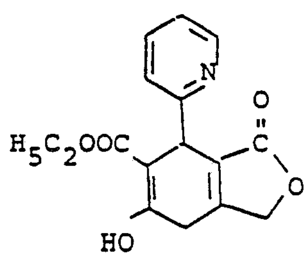

5-Hydroxy-1-oxo-7-(2-pyridyl)-1,3,4,7-tetrahydrobenzo [c] furan-6-carbonsäureethylester

Fp. : 133-135 °C.

25)

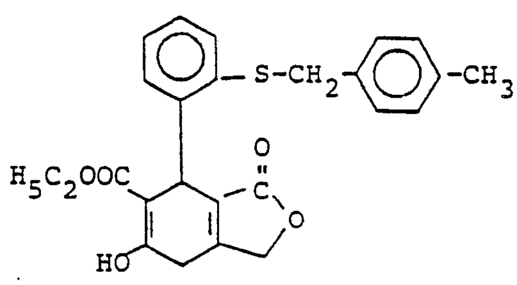

5-Hydroxy-7-(2-[4-methylbenzylthio]-phenyl)-1,3,4,7-tetrahydrobenzo [c] furan-6-carbonsäureethylester

Fp. : 130-135 °C.

**Patentansprüche**

1. Furanone der allgemeinen Formel (I)

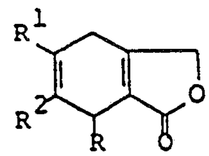

(I)

worin

R für Phenyl, Naphthyl, Pyridyl oder Thiochromenyl steht, wobei der Phenylrest gegebenenfalls durch 1 bis 3 gleiche oder verschiedene Substituenten aus der Gruppe Alkyl mit 1 bis 4 Kohlenstoffatomen, Fluor, Chlor, Nitro, Trifluormethyl, Alkoxy mit 1 bis 4 Kohlenstoffatomen, Hydroxy, Amino, Dialkylamino mit jeweils 1 bis 4 C-Atomen, Cyano, Carboxy, Carbalkoxy, Phenyl, Benzyl, Benzyloxy oder Benzylthio substituiert sein können, wobei die 4 letztgenannten Substituenten ihrerseits gegebenenfalls 1 oder 2 gleiche oder verschiedene Substituenten aus der Gruppe Alkyl, Alkoxy, Alkylthio mit jeweils 1 bis 4 C-Atomen, Fluor, Chlor, Cyano, Hydroxy, Trifluormethyl oder Nitro tragen,

$R^1$ für Hydroxyl, NH-Alkyl mit 1 bis 4 C-Atomen oder Dialkylamin mit jeweils 1 bis 4 C-Atomen in den Alkylgruppen steht und

$R^2$ für den Rest $COOR^3$ steht, wobei $R^3$ für einen geradkettigen, verzweigten oder cyclischen Alkyl- oder Alkenylrest mit bis zu 12 C-Atomen steht, der gegebenenfalls substituiert ist durch Alkoxy oder Alkylthio mit jeweils 1 bis 4 C-Atomen, Fluor, Chlor, Cyano, Hydroxy, Amino, Alkylamino oder Dialkylamino mit jeweils 1 bis 4 C-Atomen je Alkylgruppe, Nitro, Phenyl oder Pyridyl,

steht,

sowie ihre physiologisch unbedenklichen Salze.

2. Verfahren zur Herstellung von Verbindung der allgemeinen Formel (I)

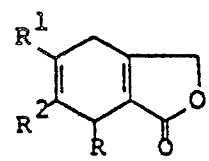

(I)

in welcher

R für Phenyl, Naphthyl, Pyridyl oder Thiochromenyl steht, wobei der Phenylrest gegebenenfalls durch 1 bis 3 gleiche oder verschiedene Substituenten aus der Gruppe Alkyl mit 1 bis 4 Kohlenstoffatomen, Fluor, Chlor, Nitro, Trifluormethyl, Alkoxy mit 1 bis 4 Kohlenstoffatomen, Hydroxy, Amino, Dialkylamino mit jeweils 1 bis 4 C-Atomen, Cyano, Carboxy, Carbalkoxy, Phenyl, Benzyl, Benzyloxy oder Benzylthio substituiert sein können, wobei die 4 letztgenannten Substituenten ihrerseits gegebenenfalls 1 oder 2 gleiche oder verschiedene Substituenten aus der Gruppe Alkyl, Alkoxy, Alkylthio mit jeweils 1 bis 4 C-Atomen, Fluor, Chlor, Cyano, Hydroxy, Trifluormethyl oder Nitro tragen,

$R^1$ für Hydroxyl, NH-Alkyl mit 1 bis 4 C-Atomen oder Dialkylamin mit jeweils 1 bis 4 C-Atomen in den Alkylgruppen steht und

$R^2$ für den Rest $COOR^3$ steht, wobei $R^3$ für einen geradkettigen, verzweigten oder cyclischen Alkyl- oder Alkenylrest mit bis zu 12 C-Atomen steht, der gegebenenfalls substituiert ist durch Alkoxy oder Alkylthio mit jeweils 1 bis 4 C-Atomen, Fluor, Chlor, Cyano, Hydroxy, Amino, Alkylamino oder Dialkylamino mit jeweils 1 bis 4 C-Atomen je Alkylgruppe, Nitro, Phenyl oder Pyridyl

steht, in denen $R^1$ nicht Hydroxyl bedeutet, dadurch gekennzeichnet, daß man

A) Aldehyde der allgemeinen Formel (II)

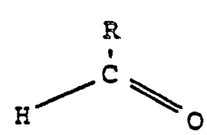

(II)

in der R die oben angegebene Bedeutung hat,
mit Verbindungen der allgemeinen Formel (III)

$$HC(R_2) = C(R_1) - CH_3$$

in der $R_1$ und $R_2$ die oben angegebenen Bedeutungen haben und Verbindungen der allgemeinen Formel (IV)

$$R^4—OOC—CH_2—CO—CH_2—O—R^5$$

in der

$R^4$ ein Alkylrest (1 bis 10 C-Atome) und

$R^5$ eine Schutzgruppe für eine OH oder SH-Gruppe darstellt, in inerten organischen Lösungsmitteln bei Temperaturen zwischen 0 °C und 120 °C umsetzt und anschließend die Schutzgruppe $R^5$ abspaltet, wobei Lactonisierung eintritt, oder

B) Benzylidenverbindungen der allgemeinen Formel (V)

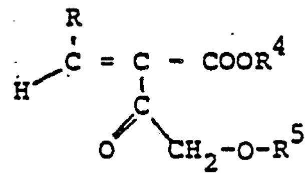

in der R, $R^4$ und $R^5$ die oben angegebenen Bedeutungen haben,
mit Verbindungen der allgemeinen Formel (III),
in der $R^1$ und $R^2$ die oben angegebenen Bedeutungen haben,
umsetzt und anschließend die Schutzgruppe $R^5$ entfernt, wobei Lactonisierung eintritt, oder

C) Benzylidenverbindungen der allgemeinen Formel (VI)

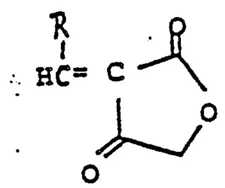

in der R die oben angegebene Bedeutung hat,
mit Verbindungen der allgemeinen Formel (III),
in der $R^1$ und $R^2$ die oben angegebene Bedeutung haben, analog A) umsetzt,
und anschließend durch Umsetzung mit wäßrigen Säuren in Anwesenheit von inerten organischen Lösungsmitteln gegebenenfalls die Hydroxylgruppe für den Substituenten $R^1$ einführt.

3. Verfahren zur Herstellung von Verbindungen der allgemeinen Formel (I) gemäß Anspruch 2, in welcher $R^1$ für Hydroxyl steht, dadurch gekennzeichnet, daß man gemäß den Verfahrensvarianten A) und/oder B) aus Anspruch 2 den Austausch der Aminogruppe durch eine Hydroxylgruppe als Eintopfreaktion durchführt.

4. Verfahren gemäß den Ansprüchen 2 und 3, dadurch gekennzeichnet, daß man die Reaktion bei Temperaturen zwischen 20 und 100° durchführt.

5. Verbindungen der allgemeinen Formel (I) gemäß Anspruch 1 zur Verwendung bei der Bekämpfung von Krankheiten.

6. Arzneimitel enthaltend mindestens eine Verbindung der allgemeinen Formel (I) gemäß Anspruch 1.

7. Verfahren zur Herstellung von Arzneimitteln, dadurch gekennzeichnet, daß man Verbindungen der allgemeinen Formel (I) gemäß Anspruch 1 gegebenenfalls unter Verwendung von inerten Hilfs- und Trägerstoffen in eine geeignete Applikationsform überführt.

8. Verwendung von Verbindungen der allgemeinen Formel (I) gemäß Anspruch 1 bei der Herstellung von kreislaufwirksamen Arzneimitteln.

## Claims

1. Furanones of the general formula (I)

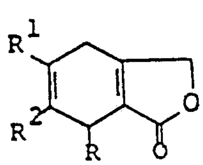

in which

R represents phenyl, naphthyl, pyridyl or thiochromenyl, it being possible for the phenyl radical

optionally to be substituted by 1 to 3 identical or different substituents from the group comprising alkyl having 1 to 4 carbon atoms, fluorine, chlorine, nitro, trifluoromethyl, alkoxy having 1 to 4 carbon atoms, hydroxyl, amino, dialkylamino having 1 to 4 C atoms in each case, cyano, carboxyl, carbalkoxy, phenyl, benzyl, benzyloxy or benzylthio, the 4 last-mentioned substituents in turn optionally bearing 1 or 2 identical or different substituents from the group comprising alkyl, alkoxy, alkylthio having 1 to 4 C atoms in each case, fluorine, chlorine, cyano, hydroxyl, trifluoromethyl or nitro,

$R^1$ represents hydroxyl, NH-alkyl having 1 to 4 C atoms or dialkylamine having 1 to 4 C atoms in each alkyl group, and

$R^2$ represents the radical $COOR^3$,

where

$R^3$ represents a straight-chain, branched or cyclic alkyl or alkenyl radical having up to 12 C atoms which is optionally substituted by alkoxy or alkylthio having 1 to 4 C atoms in each case, fluorine, chlorine, cyano, hydroxyl, amino, alkylamino or dialkylamino having 1 to 4 C atoms in each alkyl group, nitro, phenyl or pyridyl, and their physiologically acceptable salts.

2. Process for the preparation of compounds of the general formula (I)

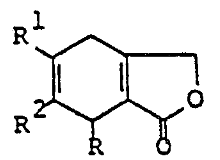

in which

R represents phenyl, naphthyl, pyridyl or thiochromenyl, it being possible for the phenyl radical optionally to be substituted by 1 to 3 identical or different substituents from the group comprising alkyl having 1 to 4 carbon atoms, fluorine, chlorine, nitro, trifluoromethyl, alkoxy having 1 to 4 carbon atoms, hydroxyl, amino, dialkylamino having 1 to 4 C atoms in each case, cyano, carboxyl, carbalkoxy, phenyl, benzyl, benzyloxy or benzylthio, the 4 last-mentioned substituents in turn optionally bearing 1 or 2 identical or different substituents from the group comprising alkyl, alkoxy, alkylthio having 1 to 4 C atoms in each case, fluorine, chlorine, cyano, hydroxyl, trifluoromethyl or nitro,

$R^1$ represents hydroxyl, NH-alkyl having 1 to 4 C atoms or dialkylamine having 1 to 4 C atoms in each alkyl group, and

$R^2$ represents the radical $COOR^3$,

where

$R^3$ represents a straight-chain, branched or cyclic alkyl or alkenyl radical having up to 12 C atoms which is optionally substituted by alkoxy or alkylthio having 1 to 4 C atoms in each case, fluorine, chlorine, cyano, hydroxyl, amino, alkylamino or dialkylamino having 1 to 4 C atoms in each alkyl group, nitro, phenyl or pyridyl,

in which $R^1$ does not denote hydroxyl, characterised in that

A) aldehydes of the general formula (II)

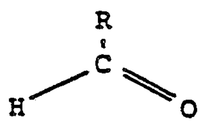

(II)

in which R has the abovementioned meaning, are reacted with compounds of the general formula (III)

$$HC(R_2) = C(R_1) - CH_3$$

in which $R_1$ and $R_2$ have the abovementioned meanings, and compounds of the general formula (IV)

$$R^4-OOC-CH_2-CO-CH_2-O-R^5$$

in which

$R^4$ represents an alkyl radical (1 to 10 C atoms), and

$R^5$ represents a protective group for an OH or SH group,

in inert organic solvents, at temperatures between 0 °C and 120 °C, and then the protective group $R^5$ is eliminated, when lactonisation occurs, or

B) benzylidene compounds of the general formula (V)

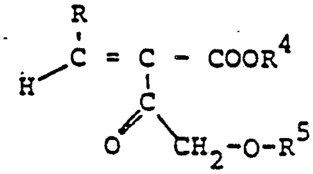

in which R, $R^4$ and $R^5$ have the abovementioned meanings, are reacted with compounds of the general formula (III), in which $R^1$ and $R^2$ have the abovementioned meanings, and then the protective group $R^5$ is removed, when lactonisation occurs, or

C) benzylidene compounds of the general formula (VI)

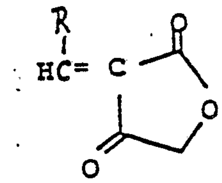

in which

R has the abovementioned meaning, are reacted with compounds of the general formula (III), in which

$R^1$ and $R^2$ have the abovementioned meaning, in analogy to A),

and then, where appropriate, the hydroxyl group is introduced for the substituent $R^1$ by reaction with aqueous acids in the presence of inert organic solvents.

3. Process for the preparation of compounds of the general formula (I) according to Claim 2, in which $R^1$ represents hydroxyl, characterised in that the exchange of the amino group by a hydroxyl group in accordance with process variants A) and/or B) from Claim 2 is carried out as a one-pot reaction.

4. Process according to Claims 2 and 3, characterised in that the reaction is carried out at temperatures between 20 and 100 °C.

5. Compounds of the general formula (I) according to Claim 1 for use in combating diseases.

6. Medicaments containing at least one compound of the general formula (I) according to Claim 1.

7. Process for the preparation of medicaments, characterised in that compounds of the general formula (I) according to Claim 1 are converted, optionally with the use of inert auxiliaries and vehicles, into a suitable form for administration.

8. Use of compounds of the general formula (I) according to Claim 1 for preparing medicaments which affect the circulation.

**Revendications**

1. Furannones de formule générale (I)

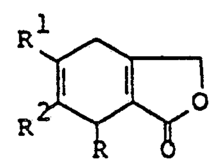

(I)

dans laquelle

R désigne un reste phényle, naphtyle, pyridyle ou thiochroményle, le reste phényle pouvant être substitué le cas échéant par 1 à 3 substituants identiques ou différents choisis dans le groupe des substituants alkyle ayant 1 à 4 atomes de carbone, fluoro, chloro, nitro, trifluorométhyle, alkoxy ayant 1 à 4 atomes de carbone, hydroxy, amino, dialkylamino avec à chaque fois 1 à 4 atomes de carbone, cyano, carboxy, carbalkoxy, phényle, benzyle, benzyloxy ou benzylthio, les quatre substituants mentionnés en dernier lieu portant quant à eux le cas échéant 1 ou 2 substituants identiques ou différents choisis dans le groupe des substituants alkyle, alkoxy, alkylthio ayant chacun 1 à 4 atomes de carbone, fluoro, chloro, cyano, hydroxy, trifluorométhyle ou nitro,

$R^1$ est un groupe hydroxyle, NH-alkyle ayant 1 à 4 atomes de carbone ou dialkylamino avec à chaque fois 1 à 4 atomes de carbone dans les groupes alkyle et

$R^2$ représente le reste $COOR^3$ dans lequel $R^3$ est un reste alkyle ou alcényle à chaîne droite, à chaîne ramifiée ou cyclique ayant jusqu'à 12 atomes de carbone, qui est éventuellement substitué par un substituant alkoxy ou alkylthio ayant chacun 1 à 4 atomes de carbone, fluoro, chloro, cyano, hydroxy, amino, alkylamino ou dialkylamino avec 1 à 4 atomes de carbone par groupe alkyle, nitro, phényle ou pyridyle, ainsi que leurs sels acceptables du point de vue physiologique.

2. Procédé de préparation d'un composé de formule générale (I)

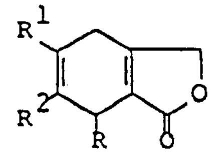

(I)

dans laquelle

R désigne un reste phényle, naphtyle, pyridyle ou thiochroményle, le reste phényle pouvant être substitué le cas échéant par 1 à 3 substituants identiques ou différents choisis dans le groupe des substituants alkyle ayant 1 à 4 atomes de carbone, fluoro, chloro, nitro, trifluorométhyle, alkoxy ayant 1 à 4 atomes de carbone, hydroxy, amino, dialkylamino avec à chaque fois 1 à 4 atomes de carbone, cyano, carboxy, carbalkoxy, phényle, benzyle, benzyloxy ou benzylthio, les quatre substituants mentionnés en dernier lieu portant quant à eux le cas échéant 1 ou 2 substituants identiques ou différents choisis dans le groupe des substituants alkyle, alkoxy, alkylthio ayant chacun 1 à 4 atomes de carbone, fluoro, chloro, cyano, hydroxy, trifluorométhyle ou nitro,

$R^1$ est un groupe hydroxyle, NH-alkyle ayant 1 à 4 atomes de carbone ou dialkylamino avec à chaque fois 1 à 4 atomes de carbone dans les groupes alkyle et

$R^2$ représente le reste $COOR^3$ dans lequel $R^3$ est un reste alkyle ou alcényle à chaîne droite, à chaîne ramifiée ou cyclique ayant jusqu'à 12 atomes de carbone, qui est éventuellement substitué par un substituant alkoxy ou alkylthio ayant chacun 1 à 4 atomes de carbone, fluoro, chloro, cyano, hydroxy, amino, alkylamino ou dialkylamino avec 1 à 4 atomes de carbone par groupe alkyle, nitro, phényle ou pyridyle, dans les cas où $R^1$ n'est pas un groupe hydroxyle, caractérisé en ce que :

A) On fait réagir des aldéhydes de formule générale (II)

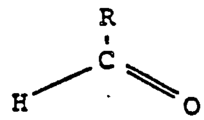

(II)

dans laquelle R a la définition indiquée ci-dessus, avec des composés de formule générale (III)

$$HC(R_2) = C(R_1) — CH_3$$

dans laquelle $R_1$ et $R_2$ ont les définitions indiquées ci-dessus et des composés de formule générale (IV)

$$R^4—OOC—CH_2—CO—CH_2—O—R_5$$

dans laquelle

$R^4$ est un reste alkyle (1 à 10 atomes de carbone) et

$R^5$ est un groupe protecteur pour un groupe OH ou SH,

dans des solvants organiques inertes, à des températures comprises entre 0 °C et 120 °C puis on élimine le groupe protecteur $R^5$, ce qui produit une lactonisation, ou bien

B) on fait réagir des composés benzylidéniques de formule générale (V)

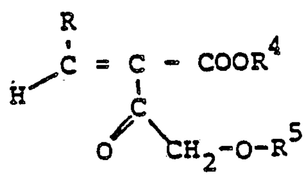

dans laquelle R, $R^4$ et $R^5$ ont les définitions indiquées ci-dessus, avec des composés de formule générale (III), dans laquelle $R^1$ et $R^2$ ont les définitions indiquées ci-dessus, puis on élimine le groupe protecteur $R^5$, et il se produit alors une lactonisation, ou bien

C) On fait réagir comme en A) des composés benzylidéniques de formule générale (VI)

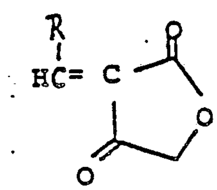

dans laquelle R a la définition indiquée ci-dessus, avec des composés de formule générale (III), dans laquelle $R^1$ et $R^2$ ont la définition indiquée ci-dessus, puis on introduit éventuellement le groupe hydroxyle pour le substituant $R^1$ par réaction avec des acides aqueux en présence de solvants organiques inertes.

3. Procédé de production de composés de formule générale (I) suivant la revendication 2, dans laquelle $R^1$ est un groupe hydroxyle, caractérisé en ce qu'on effectue l'échange du groupe amino contre un groupe hydroxyle comme réaction en un seul réacteur conformément aux variantes opératoires A) et/ou B) selon la revendication 2.

4. Procédé suivant les revendications 2 et 3, caractérisé en ce qu'on conduit la réaction à des températures comprises entre 20 et 100°.

5. Composés de formule générale (I) suivant la revendication 1 destinés à être utilisés pour combattre des maladies.

6. Médicament contenant au moins un composé de formule générale (I) suivant la revendication 1.

7. Procédé de préparation de médicaments, caractérisé en ce qu'on transforme des composés de formule générale (I) suivant la revendication 1, le cas échéant en utilisant des adjuvants et des supports inertes, en une forme d'administration appropriée.

8. Utilisation de composés de formule générale (I) suivant la revendication 1 dans la préparation de médicaments agissant sur la circulation.